# EUROPEAN PATENT APPLICATION

(11) **EP 4 692 109 A1**
(43) Date of publication of application: **11.02.2026**
(21) Application number: 24784957.3
(22) Date of filing: 04.04.2024
(51) Int. Cl.: C07K 7/08, A61K 39/215, A61P 31/14

(54) **PARTIAL PEPTIDE OF SARS-COV-2 SPIKE PROTEIN FOR INDUCING FOLLICULAR HELPER T CELLS**

(30) Priority: 07.04.2023 JP 2023062919
(71) Applicant: The University of Osaka, Osaka 565-0871 (JP)
(72) Inventor: YAMASAKI, Sho, Suita-shi, Osaka 565-0871 (JP); HAYASHI, Hiroki, Suita-shi, Osaka 565-0871 (JP); NAKAGAMI, Hironori, Suita-shi, Osaka 565-0871 (JP); LU, Xiuyuan, Suita-shi, Osaka 565-0871 (JP); ISHIKAWA, Eri, Suita-shi, Osaka 565-0871 (JP)
(74) Representative: Mewburn Ellis LLP
(86) International application number: PCT/JP2024/013937
(87) International publication number: WO 2024/210175

(57) **Abstract**

It aims to provide a composition for inducing follicular helper T cell reactive to SARS-CoV-2. A partial peptide of the spike protein of SARS-CoV-2 is provided, which contains an amino acid sequence selected from the group consisting of the following (1) to (17) and has a full length of 15 or less amino acids:
(1) FKIYSKHTPIN (SEQ ID NO: 1),
(2) FQFCNDPFLGVYYHK (SEQ ID NO: 2),
(3) KRFDNPVLPFN (SEQ ID NO: 3),
(4) LLQYGSFCTQL (SEQ ID NO: 4),
(5) PPAYTNSFTRGVYYP (SEQ ID NO: 5),
(6) CSNLLLQYGSFCTQL (SEQ ID NO: 6),
(7) SKRSFIEDLLFNKVT (SEQ ID NO: 7),
(8) TGVLTESNKKFLPFQ (SEQ ID NO: 8),
(9) TNGTKRFDNPVLPFN (SEQ ID NO: 9),
(10) NQFNSAIGKIQ (SEQ ID NO: 10),
(11) NFTISVTTEIL (SEQ ID NO: 11),
(12) STEIYQAGSTPCNGV (SEQ ID NO: 12),
(13) KVFRSSVLHST (SEQ ID NO: 13),
(14) EIRASANLAAT (SEQ ID NO: 14),
(15) NFTISVTTEILPVSM (SEQ ID NO: 15),
(16) FIKQYGDCLGDIAAR (SEQ ID NO: 16), and
(17) FIEDLLFNKVTLADA (SEQ ID NO: 17).

## Description

### [Technical Field]

The present invention relates to a partial peptide of SARS-CoV-2 spike protein, and a vaccine for inducing follicular helper T cell reactive to SARS-CoV-2, which contains the peptide.

### [Background Art]

The COVID-19 pandemic, caused by severe acute respiratory syndrome coronavirus 2 (SARS-CoV-2), is currently spreading worldwide. Many types of vaccines, including nucleic acid vaccines, inactivated vaccines, and recombinant vaccines, have been developed to date using both classical and novel technologies and are currently in the preliminary stage and clinical stage. Two messenger ribonucleic acid (mRNA) vaccines, BNT162b2/Pfizer and mRNA-1273/Moderna, each demonstrated efficacy of 90% or above in two months after the second inoculation and were approved. Pathogen-specific antibodies are generally considered the most effective factor for preventing infection. However, recent reports have shown that humoral immune responses induced by mRNA vaccines decline after six months, and vaccine efficacy actually declines over time.

Recent reports suggest that the sustainability of antibody production is related to physical factors such as gender and age. CD4+ follicular helper T (Tfh) cells have been reported to contribute to sustained antibody production. An mRNA vaccine targeting the SARS-CoV-2 spike protein strongly induces Tfh cells through germinal center (GC) responses in mice, and induces long-lived plasma cells and memory B cells. In fact, previous studies have shown that the number of Tfh cells correlates with neutralizing antibodies. In addition to humoral immune responses, the durability of memory T cells is known to be related to the suppression of deterioration or death after COVID-19-related infection. Elucidation of the critical factors involved in T cell responses is considered to provide valuable information for vaccine development hereafter.

The present inventors previously collected PBMCs from patients infected with SARS-CoV-2, stimulated them with a peptide pool derived from SARS-CoV-2, and analyzed the TCR sequences of the obtained Tfh cells, SARS-CoV-2-derived peptides that can stimulate the Tfh cells, and the HLAs that present the peptides. As a result, they reported that a specific amino acid sequence derived from SARS-CoV-2 was found that can induce Tfh cells reactive to SARS-CoV-2 from patients infected with SARS-CoV-2 (Patent Literature 1).

### [Citation List]

### [Patent Literature]

[Patent Literature 1]
WO 2022/196699 A1

### [Summary of Invention]

### [Technical Problem]

The present invention aims to find a SARS-CoV-2-derived peptide for inducing a follicular helper T cell reactive to SARS-CoV-2 from PBMC of a vaccinated patient, and provide a vaccine for inducing a follicular helper T cell reactive to SARS-CoV-2, which contains the peptide.

### [Solution to Problem]

The present inventors evaluated SARS-CoV-2-specific humoral and cellular responses, and spike protein-specific T cell clonotypes, in BNT162b2 vaccine recipients 3, 6, and 24 weeks after the first vaccine administration. As a result, antibody titers and T cell responses significantly increased 6 weeks after the first administration (3 weeks after the second administration). Although antibody titers generally decreased 24 weeks after the first administration (21 weeks after the second administration), antibody titers were relatively persistent in some test subjects. Using single-cell TCR and RNA sequencing technologies, the present inventors evaluated the relationship between sustained antibody production in peripheral blood mononuclear cells (PBMCs) and spike protein-specific T cell clonotypes. Importantly, while T cells activated by spike protein exhibit individual diversity, antibody sustainers possessed a greater number of circulating follicular helper T cell (cTfh) clonotypes compared to antibody decliners. The present inventors further determined the spike protein-derived epitopes recognized by Tfh clonotypes expanded after BNT162b2 vaccination and found that many of the epitopes were conserved or maintained antigenicity even in variants of concern (VOCs) that are circulating. These data suggest that mRNA vaccines induce various T cell responses and that spike protein-specific circulating Tfh cells may contribute to long-term antibody production after mRNA vaccination.

Based on these findings, the present inventors conducted further studies and completed the present invention.

That is, the present invention provides the following.
[1] A partial peptide of the spike protein of SARS-CoV-2, which comprises an amino acid sequence selected from the group consisting of the following (1) to (17) and has a full length of 15 or less amino acids:
   (1) FKIYSKHTPIN (SEQ ID NO: 1),
   (2) FQFCNDPFLGVYYHK (SEQ ID NO: 2),
   (3) KRFDNPVLPFN (SEQ ID NO: 3),
   (4) LLQYGSFCTQL (SEQ ID NO: 4),
   (5) PPAYTNSFTRGVYYP (SEQ ID NO: 5),
   (6) CSNLLLQYGSFCTQL (SEQ ID NO: 6),
   (7) SKRSFIEDLLFNKVT (SEQ ID NO: 7),
   (8) TGVLTESNKKFLPFQ (SEQ ID NO: 8),
   (9) TNGTKRFDNPVLPFN (SEQ ID NO: 9),
   (10) NQFNSAIGKIQ (SEQ ID NO: 10),
   (11) NFTISVTTEIL (SEQ ID NO: 11),
   (12) STEIYQAGSTPCNGV (SEQ ID NO: 12),
   (13) KVFRSSVLHST (SEQ ID NO: 13),
   (14) EIRASANLAAT (SEQ ID NO: 14),
   (15) NFTISVTTEILPVSM (SEQ ID NO: 15),
   (16) FIKQYGDCLGDIAAR (SEQ ID NO: 16), and
   (17) FIEDLLFNKVTLADA (SEQ ID NO: 17).
[2] A vaccine comprising the partial peptide of [1].
[3] The vaccine of [2], for inducing a follicular helper T cell reactive to SARS-CoV-2.
[4] The vaccine of [3], wherein the SARS-CoV-2 is a strain selected from Wuhan strain, Delta strain, and Omicron strain.
[5] The vaccine of any one of [2] to [4], which is administered to a subject selected from the group consisting of the following (1) to (17):
   (1) a vaccine administration subject having HLA gene DRB1*09:01, the vaccine comprising a partial peptide of the spike protein of SARS-CoV-2, which peptide comprises the amino acid sequence of FKIYSKHTPIN (SEQ ID NO: 1) and has a full length of 15 or less amino acids,
   (2) a vaccine administration subject having HLA genes DPA1*01:03/DPB1*04:02, the vaccine comprising a partial peptide of the spike protein of SARS-CoV-2, which peptide comprises the amino acid sequence of FQFCNDPFLGVYYHK (SEQ ID NO: 2) and has a full length of 15 or less amino acids,
   (3) a vaccine administration subject having HLA genes DPA1*02:02/DPB1*05:01, the vaccine comprising a partial peptide of the spike protein of SARS-CoV-2, which peptide comprises the amino acid sequence of KRFDNPVLPFN (SEQ ID NO: 3) and has a full length of 15 or less amino acids,
   (4) a vaccine administration subject having HLA gene DRB1*15:02, the vaccine comprising a partial peptide of the spike protein of SARS-CoV-2, which peptide comprises the amino acid sequence of LLQYGSFCTQL (SEQ ID NO: 4) and has a full length of 15 or less amino acids,
   (5) a vaccine administration subject having HLA gene DRB1*09:01, the vaccine comprising a partial peptide of the spike protein of SARS-CoV-2, which peptide comprises the amino acid sequence of PPAYTNSFTRGVYYP (SEQ ID NO: 5) and has a full length of 15 or less amino acids,
   (6) a vaccine administration subject having HLA gene DRB1*15:02, the vaccine comprising a partial peptide of the spike protein of SARS-CoV-2, which peptide comprises the amino acid sequence of CSNLLLQYGSFCTQL (SEQ ID NO: 6) and has a full length of 15 or less amino acids,
   (7) a vaccine administration subject having HLA genes DPA1*01:03/DPB1*04:02, DPA1*02:02/DPB1*04:02 or DPA1*01:03/DPB1*02:01, the vaccine comprising a partial peptide of the spike protein of SARS-CoV-2, which peptide comprises the amino acid sequence of SKRSFIEDLLFNKVT (SEQ ID NO: 7) and has a full length of 15 or less amino acids,
   (8) a vaccine administration subject having HLA gene DRB1*14:54, the vaccine comprising a partial peptide of the spike protein of SARS-CoV-2, which peptide comprises the amino acid sequence of TGVLTESNKKFLPFQ (SEQ ID NO: 8) and has a full length of 15 or less amino acids,
   (9) a vaccine administration subject having HLA genes DPA1*02:02/DPB1*05:01 or DPA1*01:03/DPB1*05:01, the vaccine comprising a partial peptide of the spike protein of SARS-CoV-2, which peptide comprises the amino acid sequence of TNGTKRFDNPVLPFN (SEQ ID NO: 9) and has a full length of 15 or less amino acids,
   (10) a vaccine administration subject having HLA gene DRB1*09:01, the vaccine comprising a partial peptide of the spike protein of SARS-CoV-2, which peptide comprises the amino acid sequence of NQFNSAIGKIQ (SEQ ID NO: 10) and has a full length of 15 or less amino acids,
   (11) a vaccine administration subject having HLA gene DRB1*09:01, the vaccine comprising a partial peptide of the spike protein of SARS-CoV-2, which peptide comprises the amino acid sequence of NFTISVTTEIL (SEQ ID NO: 11) and has a full length of 15 or less amino acids,
   (12) a vaccine administration subject having HLA gene DRB1*04:03, the vaccine comprising a partial peptide of the spike protein of SARS-CoV-2, which peptide comprises the amino acid sequence of STEIYQAGSTPCNGV (SEQ ID NO: 12) and has a full length of 15 or less amino acids,
   (13) a vaccine administration subject having HLA gene DRB1*04:03, the vaccine comprising a partial peptide of the spike protein of SARS-CoV-2, which peptide comprises the amino acid sequence of KVFRSSVLHST (SEQ ID NO: 13) and has a full length of 15 or less amino acids,
   (14) a vaccine administration subject having HLA gene DRB1*04:03, the vaccine comprising a partial peptide of the spike protein of SARS-CoV-2, which peptide comprises the amino acid sequence of EIRASANLAAT (SEQ ID NO: 14) and has a full length of 15 or less amino acids,
   (15) a vaccine administration subject having HLA gene DRB1*09:01, the vaccine comprising a partial peptide of the spike protein of SARS-CoV-2, which peptide comprises the amino acid sequence of NFTISVTTEILPVSM (SEQ ID NO: 15) and has a full length of 15 or less amino acids,
   (16) a vaccine administration subject having HLA genes DQA1*01:02/DQB1*05:03, the vaccine comprising a partial peptide of the spike protein of SARS-CoV-2, which peptide comprises the amino acid sequence of FIKQYGDCLGDIAAR (SEQ ID NO: 16) and has a full length of 15 or less amino acids, and
   (17) a vaccine administration subject having HLA genes DPA1*01:03/DPB1*02:01, the vaccine comprising a partial peptide of the spike protein of SARS-CoV-2, which peptide comprises the amino acid sequence of FIEDLLFNKVTLADA (SEQ ID NO: 17) and has a full length of 15 or less amino acids.

### [Advantageous Effects of Invention]

According to the present invention, a vaccine for inducing a follicular helper T cell reactive to SARS-CoV-2 can be provided, which contains a SARS-CoV-2-derived specific peptide found from PBMC of a vaccinated patient. The SARS-CoV-2-derived specific peptide contained in the above-mentioned vaccine actually binds to the TCR of follicular helper T cells amplified in patients who received mRNA vaccine. Thus, the administration of the peptide is expected to strongly induce follicular helper T cells. The increase in the number of follicular helper T cells contributes to the maintenance of antibody production, making it possible to induce long-term immunity.

### [Brief Description of Drawings]

[Fig. 1]

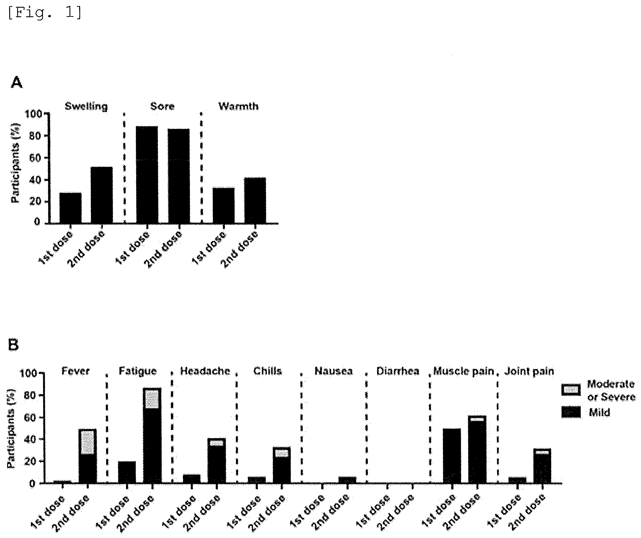

   Fig. 1 shows the scores (A) of local side effects (injection site) and the scores (B) of systemic side effects, after SARS-CoV-2 mRNA vaccine administration.
[Fig. 2]

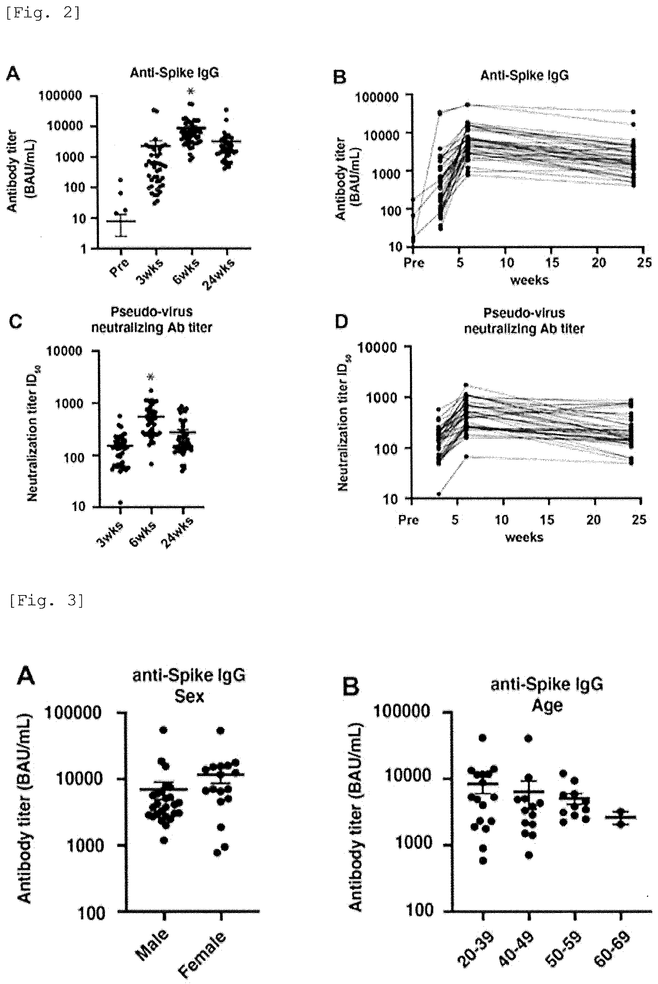

   Fig. 2(A), (B) show the anti-spike protein IgG titers of test subjects measured by ELISA after SARS-CoV-2 mRNA vaccine administration. Fig. 2(C), (D) show the ID₅₀ neutralization activity of test subjects measured by pseudo-virus assay after SARS-CoV-2 mRNA vaccine administration.
[Fig. 3]
   Fig. 3(A) shows comparison of the anti-spike protein IgG titers between men and women after SARS-CoV-2 mRNA vaccine administration. Fig. 3(B) shows comparison of the anti-spike protein IgG titers by age after SARS-CoV-2 mRNA vaccine administration.
[Fig. 4]

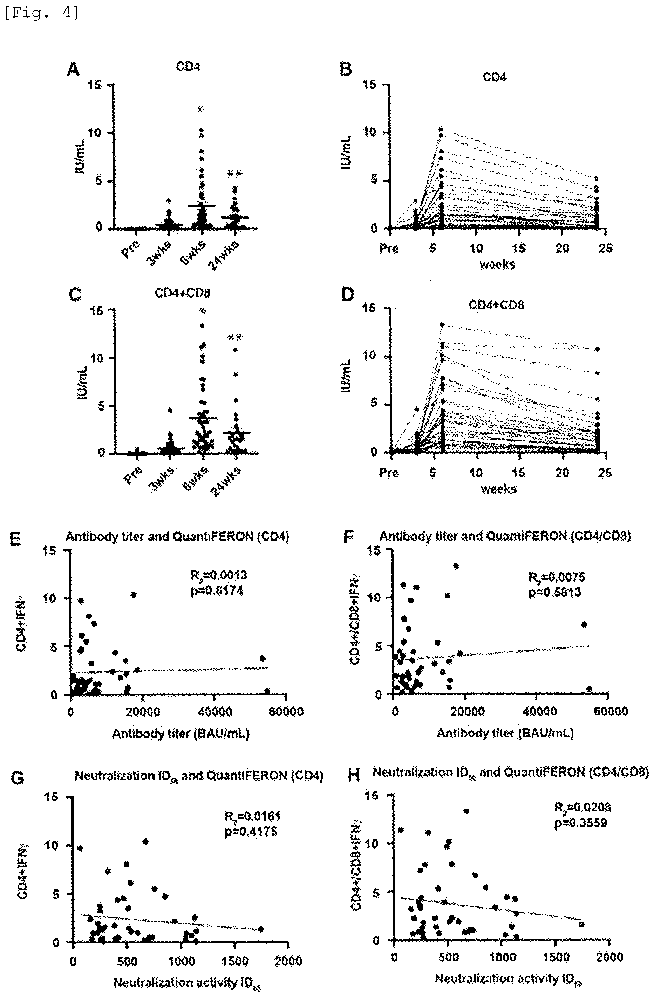

   Fig. 4(A), (B) show the amount of IFNγ released by CD4+T cells in test subjects measured using QuantiFERON after SARS-CoV-2 mRNA vaccine administration. Fig. 4(C), (D) show the amount of IFNγ released by CD4+CD8+T cells in test subjects measured using QuantiFERON after SARS-CoV-2 mRNA vaccine administration. Fig. 4(E) shows the relationship between the amount of IFNγ released by CD4+T cells and the anti-spike protein IgG titer in test subjects after SARS-CoV-2 mRNA vaccine administration. Fig. 4(F) shows the relationship between the amount of IFNγ released by CD4+CD8+T cells and the anti-spike protein IgG titer in test subjects after SARS-CoV-2 mRNA vaccine administration. Fig. 4(G) shows the relationship between the amount of IFNγ released by CD4+T cells in test subjects and the ID₅₀ neutralization activity in test subjects after SARS-CoV-2 mRNA vaccine administration. Fig. 4(H) shows the relationship between the amount of IFNγ released by CD4+CD8+T cells in test subjects and the TD₅₀ neutralization activity in test subjects after SARS-CoV-2 mRNA vaccine administration.
[Fig. 5]

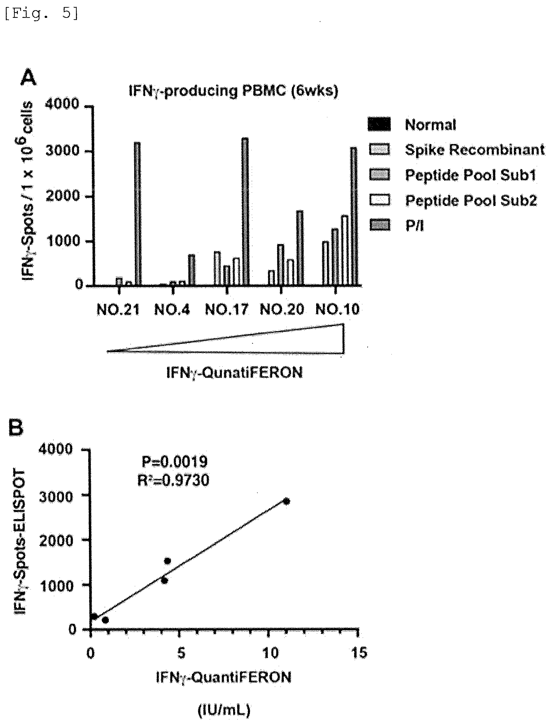

   Fig. 5(A) shows the number of spots per 1x10⁶ PBMCs after stimulating PBMCs from test subjects of SARS-CoV-2 mRNA vaccines, who showed different amounts of IFNγ release, for 48 hr with recombinant spike protein, spike protein-derived peptide pool, or phorbol 12-myristate 13-acetate (P) and ionomycin (I) as positive control. Fig. 5(B) shows the correlation between ELISPOT assay and IFNγ release amounts which was assessed using Pearson correlation coefficient.
[Fig. 6]

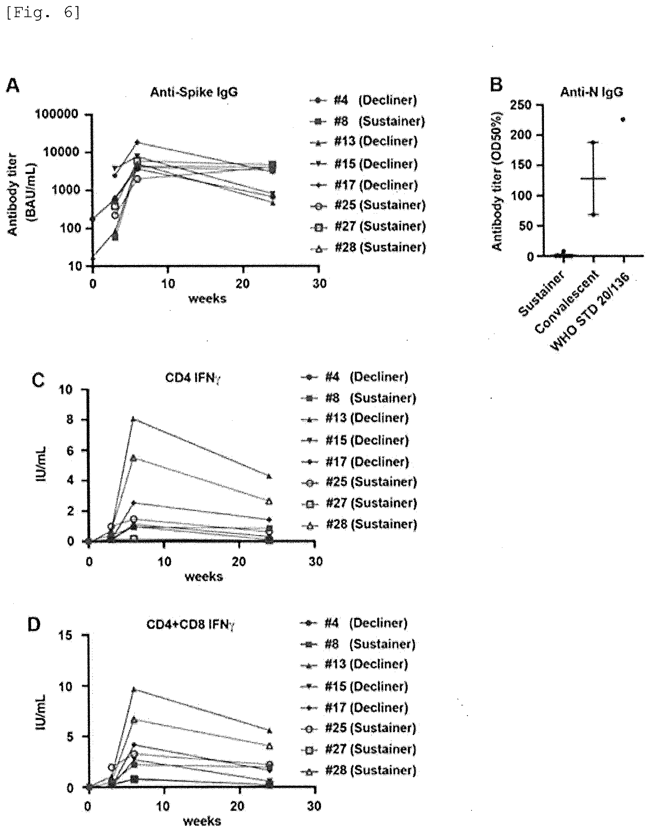

   Fig. 6(A) shows IgG titers against the anti-spike protein from 6 to 24 weeks in antibody activity sustainers and decliners. Fig. 6(B) shows IgG titer against nucleocapsid protein in sustainers. Fig. 6(C) shows the amount of IFNγ released by CD4+T cells in sustainers and decliners. Fig. 6(D) shows the amount of IFNγ released by CD4+CD8+T cells in sustainers and decliners.
[Fig. 7]

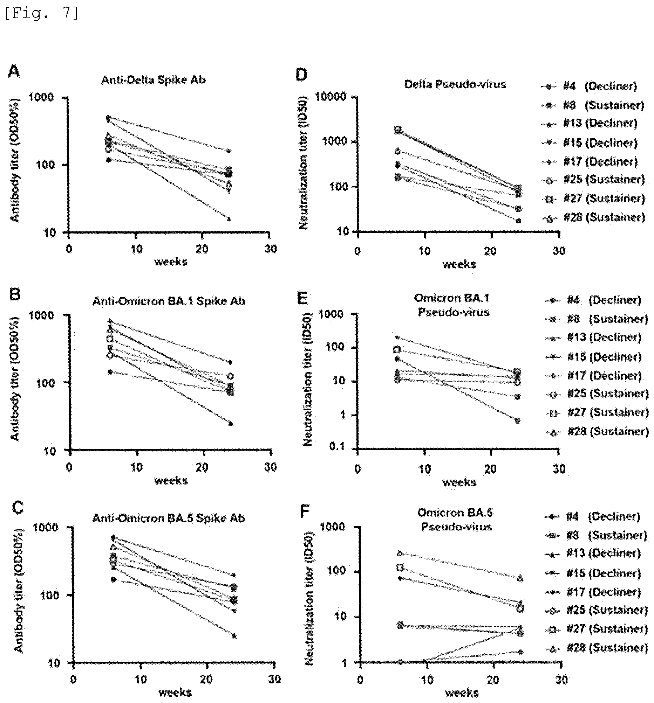

   Fig. 7 shows IgG titers against the anti-spike protein and neutralization activity against viruses from 6 to 24 weeks in sustainers and decliners. (A) Anti-Delta spike protein antibody, (B) Anti-Omicron BA.1 spike protein antibody, (C) Anti-Omicron BA.5 spike protein antibody, (D) Delta pseudo-virus, (E) Omicron BA.1 virus, (F) Omicron BA.5 virus
[Fig. 8]

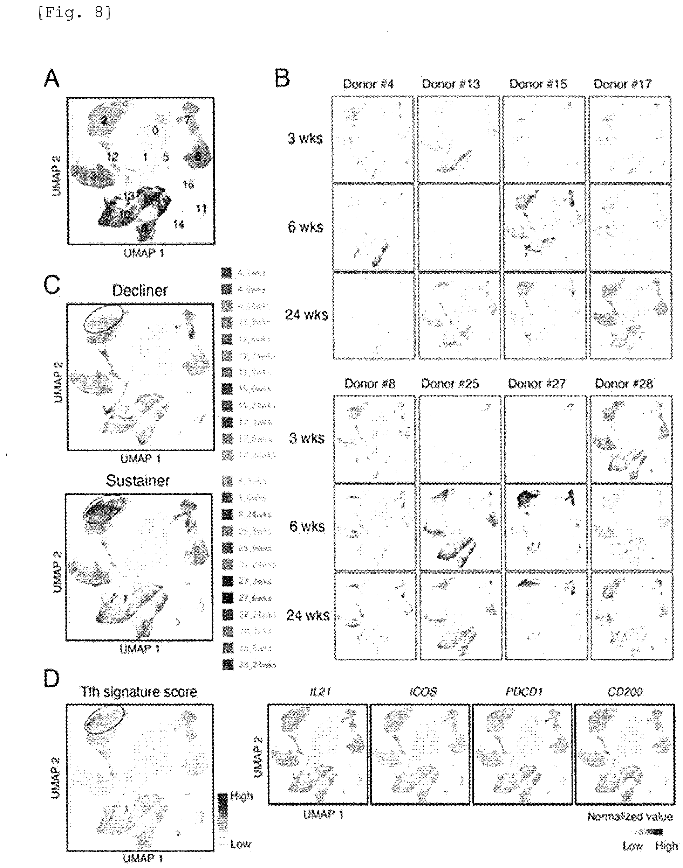

   Fig. 8(A) shows 16 clusters identified by selecting T cells stimulated and expanded with the spike peptide pool, analyzing same by single-cell TCR and RNA sequencing, and performing clustering analysis. Fig. 8(B) shows that T cells expanded by stimulation with the spike peptide pool have diversity in cell number, chronotype number, and cell type. Fig. 8(C) shows that Cluster 2 is the most distinct cell population between decliners and sustainers. Fig. 8(D) shows that cells in Cluster 2 have high Tfh scores and express genes specific to Tfh cells.
[Fig. 9]

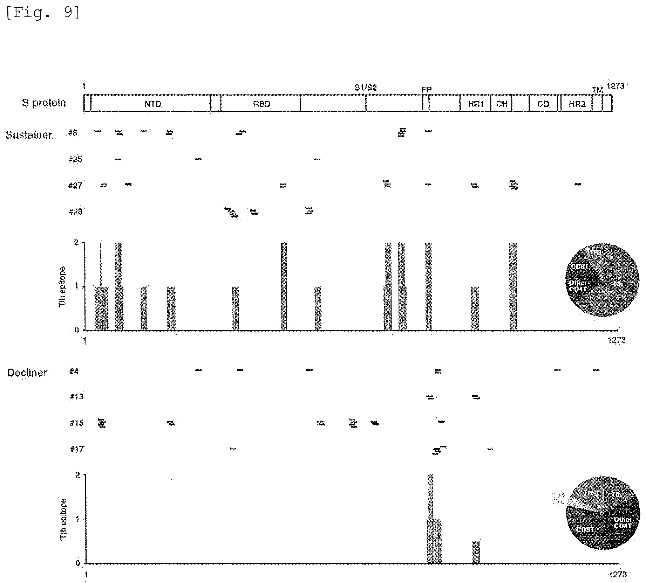

   Fig. 9 shows the location of epitopes of spike protein for TCRs of T cell clonotypes amplified from sustainers and decliners.
[Fig. 10-1]

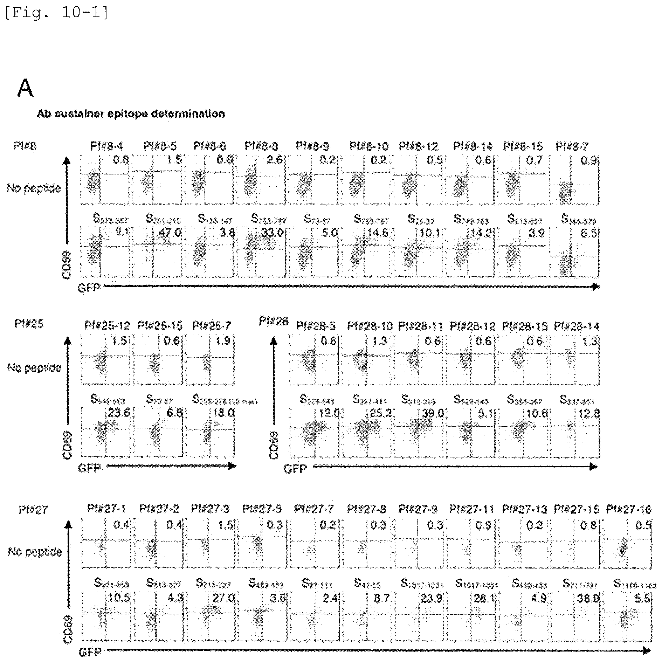

   Fig. 10-1 shows the epitope of spike proteins that can be recognized by TCR of T cell clonotypes in sustainers.
[Fig. 10-2]

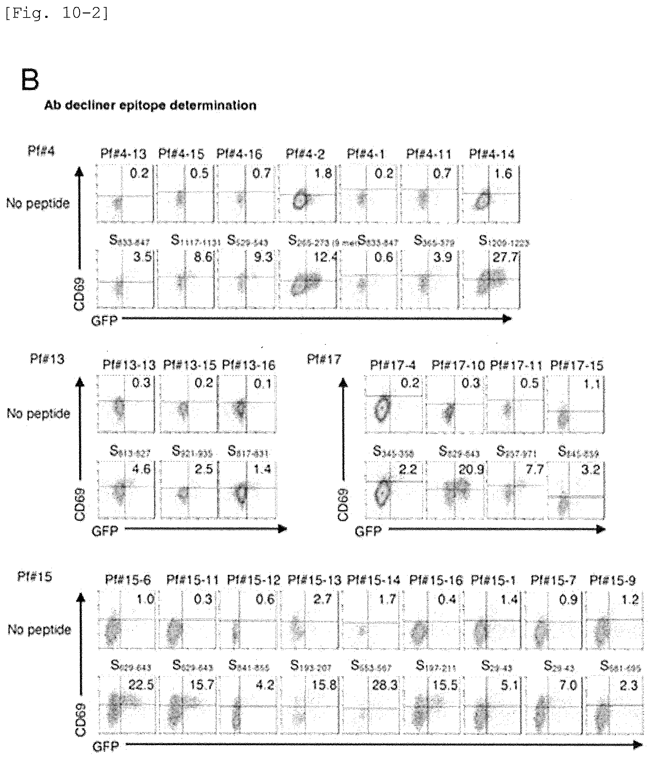

   Fig. 10-2 shows the epitope of spike proteins that can be recognized by TCR of T cell clonotypes in decliners.
[Fig. 11-1]

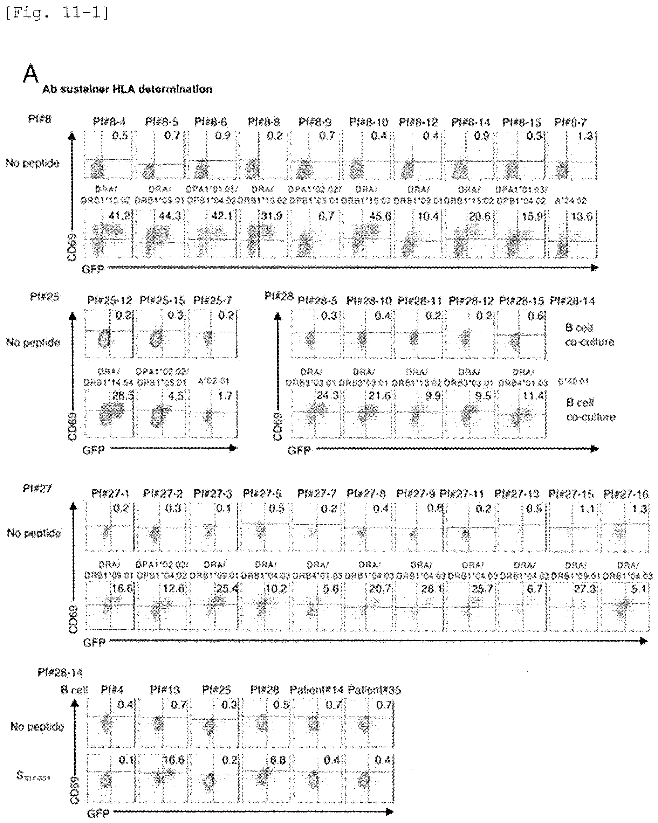

   Fig. 11-1 shows the HLAs that can be recognized by TCR of T cell clonotypes in sustainers.
[Fig. 11-2]

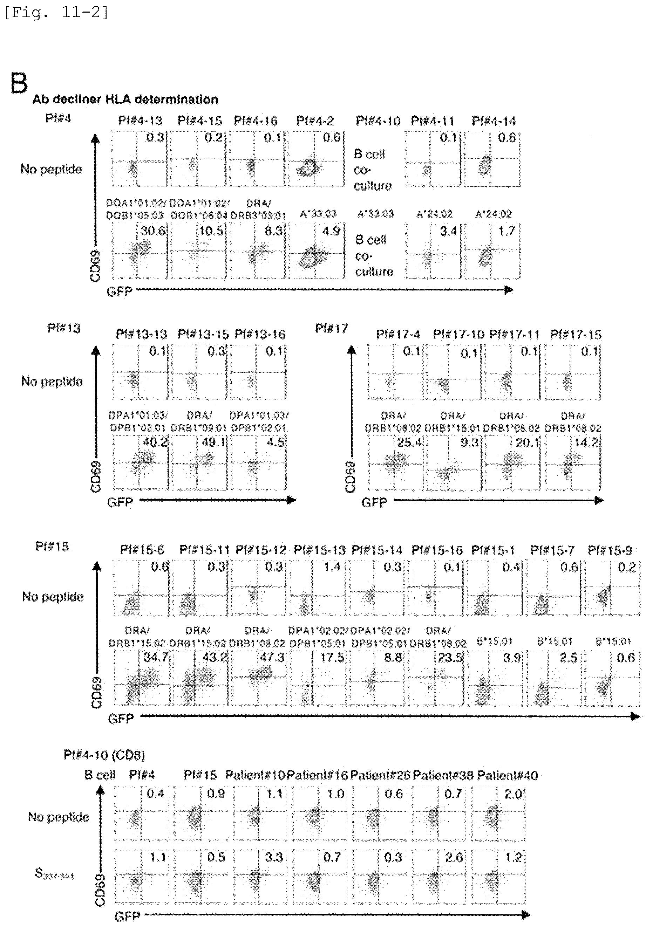

   Fig. 11-2 shows the HLAs that can be recognized by TCR of T cell clonotypes in decliners.
[Fig. 12-1]

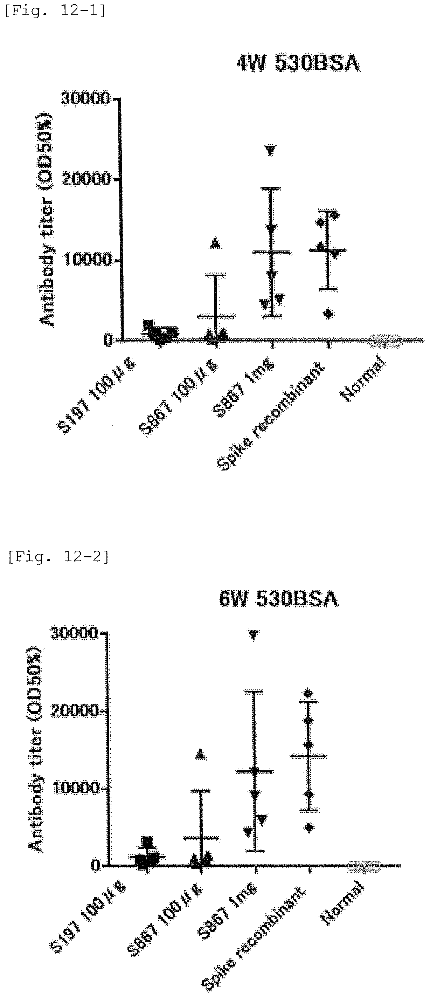

   Fig. 12-1 is a diagram showing the antibody titers in mice 4 weeks after the first vaccination.
[Fig. 12-2]
   Fig. 12-2 is a diagram showing the antibody titers in mice 6 weeks after the first vaccination.
[Fig. 12-3]

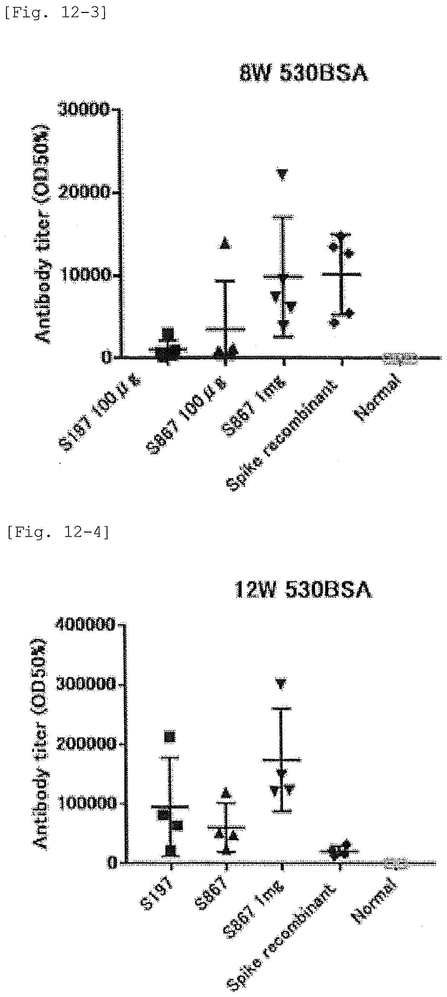

   Fig. 12-3 is a diagram showing the antibody titers in mice 8 weeks after the first vaccination.
[Fig. 12-4]
   Fig. 12-4 is a diagram showing the antibody titers in mice 12 weeks after the first vaccination.
[Fig. 12-5]

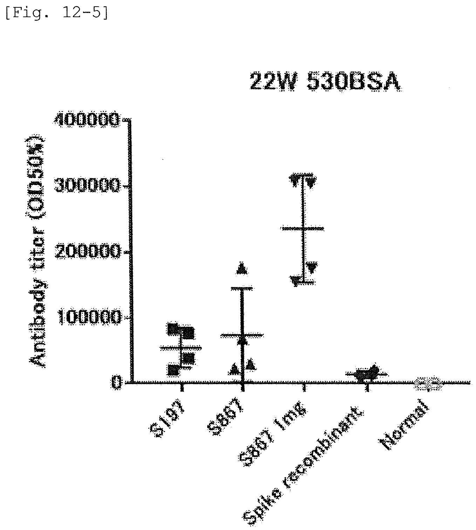

   Fig. 12-5 is a diagram showing the antibody titers in mice 22 weeks after the first vaccination.

### [Description of Embodiments]

### 1. Partial peptide of spike protein of SARS-CoV-2

The present invention provides a partial peptide of the spike protein of SARS-CoV-2 (hereinafter sometimes referred to as "the partial peptide of the present invention").

In the peptide described in the present specification, the left end is the N-terminus (amino terminus) and the right end is the C-terminus (carboxyl terminus), according to the conventional peptide notation. In the partial peptide of the present invention, the C-terminus may be any of a carboxyl group (-COOH), a carboxylate (-COO-), an amide (-CONH₂), or an ester (-COOR).

Examples of R in the ester include C₁₋₆ alkyl groups such as methyl, ethyl, n-propyl, isopropyl, n-butyl, and the like; C₃₋₈ cycloalkyl groups such as cyclopentyl, cyclohexyl, and the like; C₆₋₁₂ aryl groups such as phenyl, α-naphthyl, and the like; phenyl-C₁₋₂ alkyl groups such as benzyl, phenethyl, and the like; C₇₋₁₄ aralkyl groups such as α-naphthyl-C₁₋₂ alkyl group such as α-naphthylmethyl and the like; a pivaloyloxymethyl group, and the like.

When the partial peptide of the present invention has a carboxyl group (or carboxylate) other than that at the C-terminus, the partial peptide of the present invention also includes those in which the carboxyl group is amidated or esterified. In this case, the ester used may be, for example, the above-mentioned C-terminal ester.

Furthermore, the partial peptide of the present invention also includes those in which the amino group of the N-terminal amino acid residue is protected with a protecting group (e.g., C₁₋₆ acyl group such as formyl group and C₁₋₆ alkanoyl group such as acetyl group), those in which the amino group of the N-terminal amino acid residue is acetylated, those in which substituents on the side chains of amino acids in the molecule (e.g., -OH, -SH, amino group, imidazole group, indole group, guanidino group, etc.) are protected with appropriate protecting groups (e.g., C₁₋₆ acyl group such as formyl group and C₁₋₆ alkanoyl group such as acetyl group). In one embodiment, the partial peptide of the present invention has an acetylated amino group in the N-terminal amino acid residue and/or an amidated carboxyl group at the C-terminus.

The partial peptide of the present invention is composed of specific partial peptides of the SARS-CoV-2 spike protein. The partial peptide actually binds to the TCR of follicular helper T cells amplified in patients inoculated with mRNA vaccine. Therefore, administration of the peptide can strongly induce follicular helper T cells. Thus, administration of the partial peptides of the present invention to a subject increases the number of follicular helper T cells, and an increase in the number of follicular helper T cells contributes to the maintenance of antibody production by B cells and enables the long-term induction of neutralizing antibodies.

The spike protein of SARS-CoV-2 forms a trimer on the envelope, and each spike protein is composed of an S1 subunit and an S2 subunit. The S1 subunit and S2 subunit are generated by translation as a single protein molecule, followed by cleavage into two molecules. The S1 subunit has a receptor-binding domain (RBD) that recognizes a cell surface receptor (ACE2), and the S2 subunit contains a functional domain involved in membrane fusion.

The follicular helper T cell refers to a helper T cell that regulates B cell maturation and activation, and antibody production. It is also referred to as a follicular T cell which is used interchangeably in the present specification to mean the same as a follicular helper T cell.

As such specific partial peptide of the spike protein of SARS-CoV-2, a peptide containing an amino acid sequence selected from the group consisting of the following (1) to (17), and having a full length of 15 or less amino acids can be mentioned.
(1) FKIYSKHTPIN (SEQ ID NO: 1).
(2) FQFCNDPFLGVYYHK (SEQ ID NO: 2).
(3) KRFDNPVLPFN (SEQ ID NO: 3).
(4) LLQYGSFCTQL (SEQ ID NO: 4).
(5) PPAYTNSFTRGVYYP (SEQ ID NO: 5).
(6) CSNLLLQYGSFCTQL (SEQ ID NO: 6).
(7) SKRSFIEDLLFNKVT (SEQ ID NO: 7).
(8) TGVLTESNKKFLPFQ (SEQ ID NO: 8).
(9) TNGTKRFDNPVLPFN (SEQ ID NO: 9).
(10) NQFNSAIGKIQ (SEQ ID NO: 10).
(11) NFTISVTTEIL (SEQ ID NO: 11).
(12) STEIYQAGSTPCNGV (SEQ ID NO: 12).
(13) KVFRSSVLHST (SEQ ID NO: 13).
(14) EIRASANLAAT (SEQ ID NO: 14).
(15) NFTISVTTEILPVSM (SEQ ID NO: 15).
(16) FIKQYGDCLGDIAAR (SEQ ID NO: 16).
(17) FIEDLLFNKVTLADA (SEQ ID NO: 17).

In another embodiment of the present invention, as a specific partial peptide of the spike protein of SARS-CoV-2, a peptide containing an amino acid sequence selected from the group consisting of the following (18) and (19), and having a full length of 15 or less amino acids is provided.
(18) DGYFKIYSKHTPINL (SEQ ID NO: 69).
(19) EMIAQYTSALLAGT (SEQ ID NO: 70).

As shown in the Examples (Experimental Example 1) described later, the antibody titers of the test subjects generally decreased 24 weeks after the first BNT162b2 vaccine administration (21 weeks after the second administration) (antibody decliner), but the antibody titers were relatively sustained in some subjects (antibody sustainer). The antibody sustainer had more follicular helper T cell chronotypes as compared with antibody decliners. Furthermore, 15 partial peptides of the SARS-CoV-2 spike protein that bind to the TCR of expanded follicular helper T cells in antibody sustainers ((1) to (15) above) and four partial peptides of the SARS-CoV-2 spike protein that bind to expanded follicular helper T cells in antibody decliners ((7), (10), (16), and (17) above) were identified.

The partial peptide of the present invention which is selected from the group consisting of the above-mentioned (1) to (17) can induce neutralizing antibodies by inducing follicular helper T cells. Among the above-mentioned partial peptides, SKRSFIEDLLFNKVT (SEQ ID NO: 7) was confirmed twice in sustainers and once in decliners. In addition, NQFNSAIGKIQ (SEQ ID NO: 10) was confirmed once in sustainers and once in decliners. In addition, STEIYQAGSTPCNGV (SEQ ID NO: 12) was confirmed twice in sustainers. In addition, EIRASANLAAT (SEQ ID NO: 14) was confirmed twice in sustainers. These epitopes were commonly found at least once in the test subjects who received vaccine administration, and are expected to induce neutralizing antibodies by inducing follicular helper T cells in a wider range of subjects.

Furthermore, as described in the Example (Test Example 2) below, the partial peptides (18) and (19) have the effect of increasing mouse antibody titers.

Among the partial peptides of the present invention, FKIYSKHTPIN (SEQ ID NO: 1), KRFDNPVLPFN (SEQ ID NO: 3), LLQYGSFCTQL (SEQ ID NO: 4), NQFNSAIGKIQ (SEQ ID NO: 10), NFTISVTTEIL (SEQ ID NO: 11), KVFRSSVLHST (SEQ ID NO: 13), and EIRASANLAAT (SEQ ID NO: 14) consist of 11 amino acids. These peptides may have further amino acids added to the termini, as long as the total length is 15 amino acids or less. Examples of such partial peptide include, but are not limited to, DGYFKIYSKHTPINL (SEQ ID NO: 64) containing FKIYSKHTPIN (SEQ ID NO: 1), TNGTKRFDNPVLPFN (SEQ ID NO: 65) containing KRFDNPVLPFN (SEQ ID NO: 3), CSNLLLQYGSFCTQL (SEQ ID NO: 66) containing LLQYGSFCTQL (SEQ ID NO: 4), KVFRSSVLHSTQDLF (SEQ ID NO: 67) containing KVFRSSVLHST (SEQ ID NO: 13), EIRASANLAATKMSE (SEQ ID NO: 68) containing EIRASANLAAT (SEQ ID NO: 14), and the like.

The partial peptide of the present invention can be produced as a recombinant protein. Specifically, it can be produced by preparing a gene encoding the partial peptide of the present invention using known genetic engineering techniques, constructing a recombinant expression vector into which the target gene has been inserted so that it can be expressed, introducing this vector into a suitable host cell for expression as a recombinant protein, and purifying same. In addition, the partial peptides of the present invention can be produced by solid-phase synthesis methods (Fmoc method, Boc method) or liquid-phase synthesis method according to known general peptide synthesis protocols.

### 2. Vaccine containing partial peptide of spike protein of SARS-CoV-2

The present invention also provides a vaccine containing the partial peptide of the present invention (hereinafter sometimes referred to as "the vaccine of the present invention"). The vaccine containing the partial peptide of the present invention can induce a follicular helper T cell reactive to SARS-CoV-2.

The vaccine of the present invention may further contain a carrier protein to enhance the immunogenicity of the partial peptide of the present invention. Carrier proteins are generally substances that confer immunogenicity by binding to molecules (haptens) that do not have immunogenicity due to small molecular weights thereof, and some of them are known in the art. Preferred examples of the carrier protein include bovine serum albumin (BSA), rabbit serum albumin (RSA), ovalbumin (OVA), keyhole limpet hemocyanin (KLH), thyroglobulin (TG), diphtheria toxin (CRM197) detoxified by replacing some of the amino acids in diphtheria toxin, immunoglobulins, and the like. The carrier protein may be conjugated to the N-terminus or C-terminus of the partial peptide of the present invention. Conjugation can be achieved by introducing a cysteine residue into the partial peptide of the present invention and binding same to the amino group of a carrier protein via the SH group in the side chain of the cysteine (MBS method). Conjugation can also be achieved by binding amino groups, such as ε-amino group, α-amino group, and the like, of a lysine residue in a protein, to each other (glutaraldehyde method).

The vaccine of the present invention may also further contain an adjuvant that is pharmaceutically acceptable and compatible with the active ingredient. Adjuvants are generally substances that nonspecifically enhance immune responses of the host, and many adjuvants are known in the art. The adjuvants used in the vaccine of the present invention are not particularly limited as long as they are capable of nonspecifically enhancing the immune response. Examples include alum, CpG oligodeoxynucleotides, dsRNA, montanide, squalane, saponin, and LNP (lipid nanoparticles).

The vaccine of the present invention can be provided as a vaccine containing, in addition to the antigenic peptide of the present invention, any carrier, for example, a pharmaceutically acceptable carrier.

The pharmaceutically acceptable carrier may be selected appropriately depending on the dosage form, and include, but are not limited to, excipients such as sucrose and starch, binders such as cellulose and methylcellulose, disintegrants such as starch and carboxymethylcellulose, lubricants such as magnesium stearate, flavorings such as citric acid and menthol, preservatives such as sodium benzoate and sodium bisulfite, stabilizers such as sodium citrate, suspending agents such as methylcellulose and polyvinylpyrrolidone, dispersing agents such as surfactants, diluents such as water and physiological saline, base waxes, and the like.

The vaccine of the present invention can be administered orally or parenterally to mammals. Since the partial peptide of the present invention may be degraded in the stomach, it is preferably administered parenterally. Formulations suitable for oral administration include liquids, capsules, sachets, tablets, suspensions, emulsions, and the like. Formulations suitable for parenteral administration (e.g., subcutaneous injection, intramuscular injection, local injection, intraperitoneal administration, and the like) include aqueous and non-aqueous isotonic sterile injection solutions, which may contain antioxidants, buffers, bacteriostatic agents, isotonicity agents, and the like. In addition, aqueous and non-aqueous sterile suspensions can be mentioned, which may contain suspending agents, solubilizers, thickeners, stabilizers, preservatives, and the like. The formulations can be packaged in unit-dose or multi-dose containers, such as ampoules or vials. Alternatively, the active ingredient and a pharmaceutically acceptable carrier can be freeze-dried and stored in a state requiring only dissolving or suspending in an appropriate sterile vehicle immediately before use.

The content of the active ingredient (i.e., the partial peptide of the present invention) in the vaccine of the present invention is generally, but is not limited to, about 0.001 - 100 wt%, preferably 0.05 - 99 wt%, further preferably 0.1 - 90 wt%, of the entire composition.

The target of administration of the vaccine of the present invention is not particularly limited as long as it is a mammal capable of inducing a follicular helper T cell reactive to SARS-CoV-2. Examples of such mammal include rodents such as mouse and the like, pets such as dog and the like, livestock animals such as pig, horse, cow, and the like, and primates such as human, monkey, orangutan, chimpanzee, and the like, and humans are particularly preferred.

The dose of the vaccine of the present invention varies depending on the administration target, administration method, administration form, and the like. Generally, the partial peptide of the present invention is administered in the range of 1 µg to 300,000 µg, preferably 20 µg to 30,000 µg, per dose per adult, two to three times over a period of 4 weeks to 12 weeks. When the antibody titer decreases, an additional dose can be administered each time.

The follicular helper T cells that can be induced by the vaccine of the present invention are follicular helper T cells reactive to SARS-CoV-2. Examples of SARS-CoV-2 to which the follicular helper T cells induced by the vaccine of the present invention is reactive include Wuhan strain, Delta strain, and Omicron strain (BA.1 or BA.5).

The subject of administration of the vaccine of the present invention is not particularly limited as long as it has an HLA gene capable of presenting the partial peptide of the present invention. For example, a subject of administration selected from the group consisting of the following (1) to (17) can be mentioned.
(1) a subject of administration having DRB1*09:01 as an HLA gene capable of presenting a partial peptide of the spike protein of SARS-CoV-2, which peptide comprises the amino acid sequence of FKIYSKHTPIN (SEQ ID NO: 1) and has a full length of 15 or less amino acids,
(2) a subject of administration having DPA1*01:03/DP31*04:02 as HLA genes capable of presenting a partial peptide of the spike protein of SARS-CoV-2, which peptide comprises the amino acid sequence of FQFCNDPFLGVYYHK (SEQ ID NO: 2) and has a full length of 15 or less amino acids,
(3) a subject of administration having DPA1*02:02/DPB1*05:01 as an HLA gene capable of presenting a partial peptide of the spike protein of SARS-CoV-2, which peptide comprises the amino acid sequence of KRFDNPVLPFN (SEQ ID NO: 3) and has a full length of 15 or less amino acids,
(4) a subject of administration having DRB1*15:02 as an HLA gene capable of presenting a partial peptide of the spike protein of SARS-CoV-2, which peptide comprises the amino acid sequence of LLQYGSFCTQL (SEQ ID NO: 4) and has a full length of 15 or less amino acids,
(5) a subject of administration having DRB1*09:01 as an HLA gene capable of presenting a partial peptide of the spike protein of SARS-CoV-2, which peptide comprises the amino acid sequence of PPAYTNSFTRGVYYP (SEQ ID NO: 5) and has a full length of 15 or less amino acids,
(6) a subject of administration having DRB1*15:02 as an HLA gene capable of presenting a partial peptide of the spike protein of SARS-CoV-2, which peptide comprises the amino acid sequence of CSNLLLQYGSFCTQL (SEQ ID NO: 6) and has a full length of 15 or less amino acids,
(7) a subject of administration having DPA1*01:03/DPB1*04:02, DPA1*02:02/DPB1*04:02 or DPA1*01:03/DPB1*02:01 as HLA genes capable of presenting a partial peptide of the spike protein of SARS-CoV-2, which peptide comprises the amino acid sequence of SKRSFIEDLLFNKVT (SEQ ID NO: 7) and has a full length of 15 or less amino acids,
(8) a subject of administration having DRB1*14:54 as an HLA gene capable of presenting a partial peptide of the spike protein of SARS-CoV-2, which peptide comprises the amino acid sequence of TGVLTESNKKFLPFQ (SEQ ID NO: 8) and has a full length of 15 or less amino acids,
(9) a subject of administration having DPA1*02:02/DPB1*05:01 or DPA1*01:03/DPB1*05:01 as HLA genes capable of presenting a partial peptide of the spike protein of SARS-CoV-2, which peptide comprises the amino acid sequence of TNGTKRFDNPVLPFN (SEQ ID NO: 9) and has a full length of 15 or less amino acids,
(10) a subject of administration having DRB1*09:01 as an HLA gene capable of presenting a partial peptide of the spike protein of SARS-CoV-2, which peptide comprises the amino acid sequence of NQFNSAIGKIQ (SEQ ID NO: 10) and has a full length of 15 or less amino acids,
(11) a subject of administration having DRB1*09:01 as an HLA gene capable of presenting a partial peptide of the spike protein of SARS-CoV-2, which peptide comprises the amino acid sequence of NFTISVTTEIL (SEQ ID NO: 11) and has a full length of 15 or less amino acids,
(12) a subject of administration having DRB1*04:03 as an HLA gene capable of presenting a partial peptide of the spike protein of SARS-CoV-2, which peptide comprises the amino acid sequence of STEIYQAGSTPCNGV (SEQ ID NO: 12) and has a full length of 15 or less amino acids,
(13) a subject of administration having DRB1*04:03 as an HLA gene capable of presenting a partial peptide of the spike protein of SARS-CoV-2, which peptide comprises the amino acid sequence of KVFRSSVLHST (SEQ ID NO: 13) and has a full length of 15 or less amino acids,
(14) a subject of administration having DRB1*04:03 as an HLA gene capable of presenting a partial peptide of the spike protein of SARS-CoV-2, which peptide comprises the amino acid sequence of EIRASANLAAT (SEQ ID NO: 14) and has a full length of 15 or less amino acids,
(15) a subject of administration having DRB1*09:01 as an HLA gene capable of presenting a partial peptide of the spike protein of SARS-CoV-2, which peptide comprises the amino acid sequence of NFTISVTTEILPVSM (SEQ ID NO: 15) and has a full length of 15 or less amino acids,
(16) a subject of administration having DQA1*01:02/DQB1*05:03 as an HLA gene capable of presenting a partial peptide of the spike protein of SARS-CoV-2, which peptide comprises the amino acid sequence of FIKQYGDCLGDIAAR (SEQ ID NO: 16) and has a full length of 15 or less amino acids, and
(17) a subject of administration having DPA1*01:03/DPB1*02:01 as HLA genes capable of presenting a partial peptide of the spike protein of SARS-CoV-2, which peptide comprises the amino acid sequence of FIEDLLFNKVTLADA (SEQ ID NO: 17) and has a full length of 15 or less amino acids.

### 3. Follicular helper T cell reactive to SARS-CoV-2

The present invention provides a follicular helper T cell reactive to SARS-CoV-2 (hereinafter sometimes referred to as "the follicular helper T cell of the present invention").

The TCR of the follicular helper T cell of the present invention is not particularly limited as long as it can recognize the partial peptide of the present invention. For example, it includes a combination of TCRα chain CDR3 and TCRβ chain CDR3 selected from the group consisting of the following (1) to (23).
(1) TCRα chain CDR3 consisting of CAVLNQAGTALIF (SEQ ID NO: 18) and TCRβ chain CDR3 consisting of CASSGRPEGPQHF (SEQ ID NO: 19).
(2) TCRα chain CDR3 consisting of CAESRYMGRRALTF (SEQ ID NO: 20) and TCRβ chain CDR3 consisting of CASSLEGTEAFF (SEQ ID NO: 21).
(3) TCRα chain CDR3 consisting of CAGAGGTSYGKLTF (SEQ ID NO: 22) and TCRβ chain CDR3 consisting of CASSLLGEQYF (SEQ ID NO: 23).
(4) TCRα chain CDR3 consisting of CVVNKGSSASKIIF (SEQ ID NO: 24) and TCRβ chain CDR3 consisting of CASSEGASNQPQHF (SEQ ID NO: 25).
(5) TCRα chain CDR3 consisting of CALSMNTGFQKLVF (SEQ ID NO: 26) and TCRβ chain CDR3 consisting of CSAYSIYNEQFF (SEQ ID NO: 27).
(6) TCRα chain CDR3 consisting of CVVSIGFGNVLHC (SEQ ID NO: 28) and TCRβ chain CDR3 consisting of CASRPNRGDNSPLHF (SEQ ID NO: 29).
(7) TCRα chain CDR3 consisting of CAVRRGGSGGSNYKLTF (SEQ ID NO: 30) and TCRβ chain CDR3 consisting of CASSLMGGAYGYTF (SEQ ID NO: 31).
(8) TCRα chain CDR3 consisting of CLVGPYNQGGKLIF (SEQ ID NO: 32) and TCRβ chain CDR3 consisting of CSARDVEVGSGYTF (SEQ ID NO: 33).
(9) TCRα chain CDR3 consisting of CVVGTDSWGKLQF (SEQ ID NO: 34) and TCRβ chain CDR3 consisting of CASSPLSGSSYEQYF (SEQ ID NO: 35).
(10) TCRα chain CDR3 consisting of CALTSAAGNKLTF (SEQ ID NO: 36) and TCRβ chain CDR3 consisting of CSAIAGDADTQYF (SEQ ID NO: 37).
(11) TCRα chain CDR3 consisting of CVVSERASSYKLIF (SEQ ID NO: 38) and TCRβ chain CDR3 consisting of CAWNLGGGNQPQHF (SEQ ID NO: 39).
(12) TCRα chain CDR3 consisting of CLVGDSDTGRRALTF (SEQ ID NO: 40) and TCRβ chain CDR3 consisting of CASSQGQGSYGYTF (SEQ ID NO: 41).
(13) TCRα chain CDR3 consisting of CAVKRGNQGGKLIF (SEQ ID NO: 42) and TCRβ chain CDR3 consisting of CASGTGSYNEQFF (SEQ ID NO: 43).
(14) TCRα chain CDR3 consisting of CAVGIRGNEKLTF (SEQ ID NO: 44) and TCRβ chain CDR3 consisting of CASSYSGGTVTGELFF (SEQ ID NO: 45).
(15) TCRα chain CDR3 consisting of CATNAGGTSYGKLTF (SEQ ID NO: 46) and TCRβ chain CDR3 consisting of CSARDGQTATNEKLFF (SEQ ID NO: 47).
(16) TCRα chain CDR3 consisting of CALGSTSNTGKLIF (SEQ ID NO: 48) and TCRβ chain CDR3 consisting of CAWSVKGFPSQHF (SEQ ID NO: 49).
(17) TCRα chain CDR3 consisting of CAGAKGSGTYKYIF (SEQ ID NO: 50) and TCRβ chain CDR3 consisting of CASSSRTGYNSPLHF (SEQ ID NO: 51).
(18) TCRα chain CDR3 consisting of CVVNMVTGGYNKLIF (SEQ ID NO: 52) and TCRβ chain CDR3 consisting of CASSSDRNYGYTF (SEQ ID NO: 53).
(19) TCRα chain CDR3 consisting of CASSYPPSGGRTGFGEAFF (SEQ ID NO: 54) and TCRβ chain CDR3 consisting of CAMRDIGFGNVLHC (SEQ ID NO: 55).
(20) TCRα chain CDR3 consisting of CAASIPNSGYALNF (SEQ ID NO: 56) and TCRβ chain CDR3 consisting of CSASIEQGDLGYTF (SEQ ID NO: 57).
(21) TCRα chain CDR3 consisting of CAVNEYSGAGSYQLTF (SEQ ID NO: 58) and TCRβ chain CDR3 consisting of CASSVGQSKGKSAETQYF (SEQ ID NO: 59).
(22) TCRα chain CDR3 consisting of CALSDGAGNKLTF (SEQ ID NO: 60) and TCRβ chain CDR3 consisting of CSAGDTASTYGYTF (SEQ ID NO: 61).
(23) TCRα chain CDR3 consisting of CAFMKQRGGSEKLVF (SEQ ID NO: 62) and TCRβ chain CDR3 consisting of CAWSLQGQRPQHF (SEQ ID NO: 63).

As shown in the Examples described later, combinations of CDR3 of TCRα chain and CDR3 of TCRβ chain of follicular helper T cell expanded in antibody sustainers ((1) to (19) above) and combinations of CDR3 of TCRα chain and CDR3 of TCRβ chain of follicular helper T cell expanded in antibody decliners ((20) to (23) above) were specified. Follicular helper T cells containing TCR in the combinations of CDR3 of TCRα chain and CDR3 of TCRβ chain selected from the group consisting of the above-mentioned (1) to (23) can induce neutralizing antibodies.

### 4. Pharmaceutical composition containing follicular helper T cell reactive to SARS-CoV-2

The present invention also provides a pharmaceutical composition containing the follicular helper T cell of the present invention (hereinafter sometimes referred to as "the pharmaceutical composition of the present invention"). The pharmaceutical composition containing the follicular helper T cell of the present invention can enhance acquisition of immunity to SARS-CoV-2.

The follicular helper T cell of the present invention is a follicular helper T cell reactive to SARS-CoV-2. Examples of the SARS-CoV-2 to which the follicular helper T cell is reactive include Wuhan strain, Delta strain, and Omicron strain (BA.1 or BA.5).

The pharmaceutical composition of the present invention can be administered parenterally to mammals. Formulations suitable for parenteral administration (e.g., subcutaneous injection, intramuscular injection, local injection, intraperitoneal administration, and the like) include aqueous and non-aqueous isotonic sterile injection solutions, which may contain antioxidants, buffers, bacteriostatic agents, isotonicity agents, and the like. In addition, aqueous and non-aqueous sterile suspensions can be mentioned, which may contain suspending agents, solubilizers, thickeners, stabilizers, preservatives, and the like. The formulations can be packaged in unit-dose or multi-dose containers, such as ampoules or vials.

The content of the active ingredient (i.e., the follicular helper T cell of the present invention) in the pharmaceutical composition of the present invention is generally, but is not limited to, about 1.0x10 - 1.0x10⁶ cells, preferably 1.0x10² - 1.0x10⁵ cells, further preferably 1.0x10³ - 1.0x10⁴ cells, in the composition.

The target of administration of the pharmaceutical composition of the present invention is not particularly limited as long as it is a mammal capable of enhancing the acquisition of immunity to SARS-CoV-2 by a follicular helper T cell reactive to SARS-CoV-2. Examples of such mammal include rodents such as mouse and the like, pets such as dog and the like, livestock animals such as pig, horse, cow, and the like, and primates such as human, monkey, orangutan, chimpanzee, and the like, and humans are particularly preferred.

The dose of the pharmaceutical composition of the present invention varies depending on the administration target, administration method, administration form, and the like. Generally, the follicular helper T cell of the present invention is administered in the range of 1.0x10 - 1.0x10⁶ cells, preferably 1.0x10² - 1.0x10⁵ cells, per dose per adult, two to three times over a period of 4 weeks to 12 weeks. When the antibody titer decreases, an additional dose can be administered each time.

The present invention is explained further specifically in the following Examples; however, the present invention is not limited at all by these Examples.

### [Example]

### [Experimental Example 1]

### (Material and method)

### Ethics and sample collection

This project was approved by the Osaka University Institutional Review Board (IRB): Reference Number 160106. Forty-four volunteers enrolled in this protocol, and all volunteers provided informed consent before the first blood samples were collected. Samples (serum, whole blood, and peripheral blood mononuclear cells (PBMCs)) were collected four times: 0-7 days before the first dose vaccine administration as a pre-vaccination sample, 14-21 days after the first dose vaccination as a 3-week sample, 35-49 days after the first dose inoculation as a 6-week sample, and 154-182 days after the first dose inoculation as a 24-week sample. Adverse event information was also collected from all participants simultaneously with the blood collection. PBMCs were isolated using BD vacutainer CPT^{™} cell separation tube (manufactured by Beckton Dickinson) according to the manufacturer's instructions. Isolated PBMCs were stored in liquid nitrogen until use.

### Antibody titer measurement by enzyme-linked immunosorbent assay (ELISA)

To quantify antibody production after vaccine injection, an ELISA assay was used. Briefly, recombinant precursor spike protein (S1+S2, Cell Signaling Technology; 1 µg/ml), recombinant nucleocapsid protein (Acrobiosystems; 1 µg/ml), Delta spike protein, and Omicron (BA.1, BA.5) spike protein (Acrobiosystems; 1 µg/ml) were coated onto 96-well plates overnight at 4°C. On day 2, wells were blocked with a blocking buffer (PBS-T (0.05%) Tween 20, containing 5% skim milk) for 2 hr at room temperature. Serum was diluted 10- to 31,250-fold with the blocking buffer and incubated in the wells overnight at 4°C. The next day, the wells were washed and incubated with horseradish peroxidase (HRP)-conjugated antibody (GE Healthcare) for 3 hr at room temperature. After washing the wells with PBS-T, the wells were incubated with the peroxidase chromogenic substrate 3,3'-5,5'-tetramethyl benzidine (Sigma-Aldrich) for 30 min at room temperature, and the reaction was quenched by adding 0.5N sulfuric acid (Sigma-Aldrich). The absorbance of the wells was immediately measured at 450 nm using a microplate reader (Bio-Rad). The half-maximum antibody titer for each sample was calculated from the highest absorbance within the dilution range using Prism 8 software. The calculated antibody titer was converted to BAU/mL using the WHO International Standard 20/13687 (NIBSC) for precursor spike protein-specific antibody titers.

### Whole blood interferon-γ release immunoassay (IGRA) for SARS-CoV-2-specific T cell response using QuantiFERON

SARS-CoV-2-specific T cell immune response was evaluated using QuantiFERON SARS-CoV-2 (Qiagen) according to the manufacturer's protocol (Jaganathan, S., et al. (2021), Infect Dis Ther 10, 2765-2776). CD4+ T cells were activated by the CD4+ epitope coated on the Ag1 tube, and CD4+ and CD8+ T cells were activated by the CD4+ and CD8+ T cell epitopes coated on the Ag2 tube. Briefly, 1 ml of heparinized whole blood sample was added to each of the Nil (negative control), Mito (positive control), Ag1 (CD4+), and Ag2 (CD4+ and CD8+) tubes and incubated at 37°C for 22-24 hr. The tubes were centrifuged at 3000xg for 15 min, and plasma samples were collected. IFNγ derived from activated T cells was measured using an enzyme-linked immunosorbent assay (ELISA) (manufactured by Qiangen). The IFNγ concentration in the Nil tube (negative control) was subtracted from the IFNγ concentration (IU/ml) of Ag1 or Ag2.

### Enzyme-linked immunosorbent spot (ELISpot) assay using peripheral blood mononuclear cell (PBMC)

The SARS-CoV-2-specific cellular immune response of peripheral blood mononuclear cells (PBMC) derived from vaccine recipients was measured using enzyme-linked immunosorbent spot (ELISpot). For separation of PBMC, venous blood was collected using a BD vacutainer CPT tube and centrifuged at 1,500xg for 15 min at room temperature. After removing the upper plasma layer, the PBMC layer was collected and washed with PBS by centrifugation at 300xg for 10 min. The PBMC pellet was reconstituted in Cellbanker (MITSUWA FRONTECH CORP., Japan) and stored until use. ELISpot assay to detect IFNγ/IL4 production by SARS-CoV-2-specific T cell activation was performed according to the manufacturer's protocol (Cellular Technology Limited, USA). Activated PVDF membrane 96-well plates were coated with anti-human IFNγ/IL4 capture antibody and incubated overnight at 4°C. After washing with PBS, PBMCs were adjusted to 2×10⁵ per well and stimulated with SARS-CoV-2 spike peptide pools S1 and S2 (JPT Peptide Technologies) or recombinant SARS-CoV-2 spike protein (Cell Signaling Technology) for 48 hr at 37°C. After washing with PBS, the wells were incubated with anti-human IFNγ/IL4 detection antibody for 2 hr at room temperature. IFNγ/IL4 spots were detected and counted using an ImmunoSpot S6 analyzer (Cellular Technology Ltd).

### Pseudo-virus Neutralization Assay

The neutralization activity of vaccine-induced antibody was analyzed using pseudo-virus VSV (Yoshida, S., et al. (2021). Sci Rep 11, 5934). Briefly, Vero E6 cells stably expressing TMPRSS2 were seeded into a 96-well plate and cultured at 37°C for 24 hr. Pseudo-virus was incubated with serial dilutions of inactivated rat serum at 37°C for 1 hr and then added to the Vero E6 cells. At 24 hr after infection, the cells were lysed with cell culture lysis reagent (Promega), and luciferase activity was measured using Centro XS3 LB 960 (Berthold). For neutralization activity against VOC (delta, BA.1, BA.5), SARS-CoV-2 pseudo-virus having Delta spike protein (GenBank: QWK65230.1), Omicron BA.1 spike protein (GenBank: UGN73932.1), or BA.5 spike protein (GenBank: UTZ18966.1) was constructed and used for the measurement.

### In vitro stimulation of PBMC

Cryopreserved PBMCs were thawed and washed with warmed RPMI 1640 medium (Sigma) supplemented with 5% human AB serum (GeminiBio), penicillin (Sigma), streptomycin (MP Biomedicals), and 2-mercaptoethanol (Nacalai Tesque). PBMCs were labeled with a Cell Proliferation Kit (CellTrace^{™} Violet, ThermoFisher) according to the manufacturer's protocol and stimulated with a spike peptide pool (1 µg/ml each peptide, JPT) for 10 days in the same medium, supplemented with human recombinant IL-2 (1 ng/ml, Peprotech), IL-7 (5 ng/ml, BioLegend), and IL-15 (5 ng/ml, Peprotech) on days 2, 5, and 8 of culture. On day 10, the cells were washed and stained with anti-human CD3 and TotalSeq-C hashtag antibodies. Expanded CD3⁺ T cells were sorted using a cell sorter SH800S (SONY) and used for single-cell TCR and RNA sequencing.

### Transcriptome and TCR repertoire analysis using single cell

Single-cell library was prepared using 10xGenomics reagents according to the manufacturer's protocol. After reverse transcription, cDNA was amplified for 14 cycles, and up to 50 ng of cDNA was used to construct gene expression library and TCR library. Libraries were sequenced in paired-end mode, and raw reads were processed with Cell Ranger 3.1.0 (10x Genomics). Doublets and null events were removed by gating out events in which the main hashtag reads accounted for less than 95% of the total hashtag reads using Scrublet (Wolock, S.L., et al. (2019). Cell Syst 8, 281-291). The top 4000 highly variable genes were used for clustering. Tfh scores were generated using well-known Tfh marker genes (IL21, ICOS, CD200, PDCD1, POU2AF1, BTLA, CXCR5, and CXCL13), and UMAP plots were exported using BBrowser (Le, T., et al. (2020). bioRxiv).

### Establishment and stimulation of reporter cell

TCR α and β chain cDNA sequences were introduced using retroviral vectors into a murine T cell hybridoma lacking TCR and having a nuclear factor of activated T cells (NFAT)-green fluorescent protein (GFP) reporter gene (Matsumoto, Y., et al. (2021). Biochem Biophys Res Commun 534, 680-686.). Cells with reconstituted TCR were co-cultured with 1 µg/ml of peptide in the presence of immortalized autologous B cells as antigen-presenting cells (APCs). After 20 hr, cell activation was evaluated by GFP and CD69 expression.

### Antigen-presenting cell

Transformed B cells and transfected HEK293T cells were generated for use as APCs (Lu, X., et al. (2021). J Exp Med 218.). Briefly, 3×10⁵ PBMCs were incubated with a recombinant Epstein-Barr virus (EBV) suspension (Kanda, T., et al. (2015). J Virol 89, 2684-2697.) at 37°C for 1 hr with gentle shaking every 15 min. Infected cells were cultured in RPMI 1640 medium supplemented with 20% fetal bovine serum (FBS, CAPRICORN SCIENTIFIC GmbH) containing cyclosporine A (CsA, 0.1 ug/ml, Cayman Chemical). Immortalized B lymphoblastoid cell lines were obtained after 3 weeks of culture and used as APCs. HEK 293T cells were transfected with a plasmid encoding an HLA class I/II allele (Jiang, Y., et al. (2013). T Int Immunol 25, 235-246.) to generate specific HLA-expressing APC.

### Determination of epitope and restrictive HLA

15-mer Peptides were synthesized (GenScript) to cover the entire length of the SARS-CoV-2 spike protein, in which each peptide overlaps by 11 amino acids by shifting by 4 amino acids. Each peptide was dissolved in DMSO at 12 mg/ml, and 26 semipools were prepared by mixing 12 to 15 peptides. Reporter cells with reconstituted TCR were stimulated with 1 µg/ml of a spike peptide pool (1 µg/ml per peptide, JPT), followed by 36 peptide pools consisting of three semipools, semipools, and 12 individual peptides in the presence of autologous B cells for identification of epitope peptide. In order to determine restrictive HLA, reporter cells were co-cultured with autologous and various xenogeneic B cells in the presence of 1 µg/ml of epitope peptide to narrow down the HLAs. HLA shared by activatable B cells was introduced into HEK239T cells and further co-cultured to identify the restrictive HLA.

### Statistical analysis

All values are shown as mean±SEM. Significant differences in each experiment were evaluated using one-way analysis of variance followed by Tukey's post-hoc multiple comparison test using Prism 8 software (GraphPad Software). Difference was considered significant when p-value was less than 0.05.

### (Results)

### Participants and study protocol

In this Example, a total of 43 participants were enrolled and received two times of inoculation of the SARS-CoV-2 mRNA vaccine (BNT162b2). In this Example, all participants provided informed consent and completed a questionnaire before blood sample collection. Blood samples were collected according to the approved protocol before the first SARS-CoV-2 mRNA vaccine administration, and 3, 6, and 24 weeks after the first administration. Nine out of the 43 participants were not sampled before the first vaccine administration, and one of the 43 participants was not sampled 3 weeks after the first vaccine administration, due to protocol timeframe.

### SARS-CoV-2 mRNA vaccine induces strong humoral immune responses, but the responses decline by week 24

Similar to previous reports (Polack, F.P., et al. (2020). N Engl J Med 383, 2603-2615.), most participants experienced severe local (Fig. 1A) and systemic (Fig. 1B) side effects by the second administration compared with the first administration. Three weeks after the first mRNA vaccine administration, anti-spike protein IgG antibody increased in most participants (mean: 2290.4 BAU/ml, median: 480.7 BAU/ml). Six weeks later (3 weeks after the second administration), anti-spike protein antibody titer peaked (mean: 8800.7 BAU/ml, median: 5101.7 BAU/ml). Spike protein antibody titer gradually decreased over 24 weeks (mean: 3237.5, median: 1688.7; Fig. 2A). The average rate of antibody titer decrease was 56.8% (median: 65.5%) (Fig. 2B). Women tended to have higher anti-spike protein antibody titer than men (Fig. 3A). Younger participants had higher antibody titers than older participants (Fig. 3B). The neutralization activity of the serum after vaccination was evaluated using a pseudo-virus assay (VSV). After the second vaccination, the serum showed effective neutralization activity (mean ID₅₀: mean 550.2; Fig. 2C-D). After 24 weeks, the neutralization activity (ID₅₀) decreased to a mean of 276.9. Thus, it was found that mRNA vaccine effectively activates humoral immune responses in healthy subjects, but that this activity declines with time over 24 weeks.

### SARS-CoV-2 mRNA vaccine activates cellular immune responses, but the responses decline over 24 weeks after vaccination

Cellular immune responses were evaluated using QuantiFERON. IFNγ released from CD4+T cells (Ag1) or CD4+/CD8+T cells (Ag2) elevated, though not significantly, in some participants 3 weeks after the first administration (CD4+: mean 0.415 IU/ml, median 0.197 IU/ml, CD4+/CD8+: mean 0.606 IU/ml, median 0.381 IU/ml). Three weeks after the second administration (6 weeks), IFNγ release mediated by CD4+ and CD4+/CD8+ both increased significantly (CD4+: mean 2.36 IU/ml, median 1.315 IU/ml, CD4+/CD8+: mean 3.701 IU/ml, median 2.252 IU/ml). By 24 weeks after the first administration (21 weeks after the second administration), T cell-mediated IFNγ had declined (CD4+: mean 0.980 IU/ml, median 0.406 IU/ml, CD4+/CD8+: mean 1.819IU/ml, median 0.871 IU/ml) (Fig. 4A-D). The mean reduction rate of IFNγ release was 53.4% and 51.7% (median: 61.1% and 65.6%) for CD4+ and CD4+/CD8+ T cells, respectively. QuantiFERON (CD4+ and CD4+/CD8+) values did not correlate with spike protein IgG titer or neutralizing ID₅₀ (Fig. 4E-H). CD4+/CD8+ T cell responses in PBMCs selected from low to high IGRA titers were also measured by ELISpot assay. The results of IFNy-spot were cross-referenced with IGRA results (Fig. 5A-B). Importantly, SARS-CoV-2 spike protein-specific T cell activation remained at a higher level even 24 weeks after the first vaccination, compared to 3 weeks after the vaccination.

### Spike protein-reactive T cells in donors who received vaccination are diverse

In order to investigate the role of T cells in sustaining the antibody titer, the present inventors analyzed spike protein antigen-reactive T cells in eight donors after vaccination. Four of the donors maintained anti-spike protein antibody titers for 6 to 24 weeks (sustainer donor #8, #25, #27 and #28, male participants age 49±5.2 years). On the other hand, the remaining four donors showed a drastic decrease in anti-spike protein antibody titer (decliner donors #4, #13, #15, and #17; male participants, age 48.5±10.8 years) (Fig. 6A). Analysis of N antibody titers denied the possibility of SARS-CoV-2 infection in anti-spike protein antibody sustainers (Fig. 6B). The sustainability of antibody was not related to the duration of cellular immune responses measured by QunatiFERON (Fig. 6C-D). Furthermore, VOC (Delta, Omicron, BA.1, BA.5)-specific antibody titer and neutralization activity tended to persist for 24 weeks in sustainers (Fig. 7A-F).

In order to enrich spike protein-reactive T cells, the present inventors labeled PBMC with a cell proliferation tracer and stimulated them in a spike peptide pool for 10 days. Proliferated T cells were selected and analyzed by single-cell TCR and RNA sequencing. All samples from eight donors at three time points were pooled and subjected to clustering analysis to identify 16 clusters (Fig. 8A). By comparison of the samples from different time points set vertically and the samples from different donors set horizontally, it was found that T cells proliferated by spike peptide pool stimulation show diversity in cell number, clonotype number, and cell type (Fig. 8B).

### Spike protein-reactive Tfh cell may contribute to persistence of antibody titer

When cells from decliners and sustainers were grouped, the most distinct population among the two types of donors was found to be present in Cluster 2 (Fig. 8C). These cells exhibited high Tfh scores and expressed genes characteristic of Tfh cells (Fig. 8D). Given that they were separated from PBMC, the possibility was suggested that are circulating Tfh cells (cTfh).

The present inventors selected the top 16 clonotypes with the highest cell counts (top expanded clonotypes) from each donor and analyzed the cell type by the expression of characteristic genes. Sustainers had more Tfh clonotypes than decliners, and each sustainer had at least one Tfh clonotype among the top expanded clonotypes, whereas two decliners did not have a Tfh clonotype among the top expanded clonotypes.

### Identification of spike protein antigen that expanded T cell chronotype

In order to elucidate the epitopes of highly expanded T cell clonotypes, the present inventors reconstituted TCR of T cell clonotypes in mouse T cell hybridomas lacking endogenous TCR and carrying an NFAT-GFP reporter gene, and stimulated the cell lines thereof with spike peptides. Transformed autologous B cells were used as antigen-presenting cells (APCs). Of the 27 types of spike protein epitopes recognized by expanded T cells in sustainers, 15 types of spike protein epitopes were recognized by Tfh cells. Of the 16 types of spike protein epitopes recognized by expanded T cells in decliners, 4 types of spike protein epitopes were recognized by Tfh cells (Fig. 9, Table 1, Table 2, Figs. 10-1, 10-2, 11-1, 11-2). In particular, Tfh cells were able to proliferate with epitopes present in all domains of the spike protein (Fig. 9). In SARS-CoV-2 Delta or Omicron strains, several epitopes were mutated, and some of the mutated epitopes lost the antigenicity to responsive TCR.

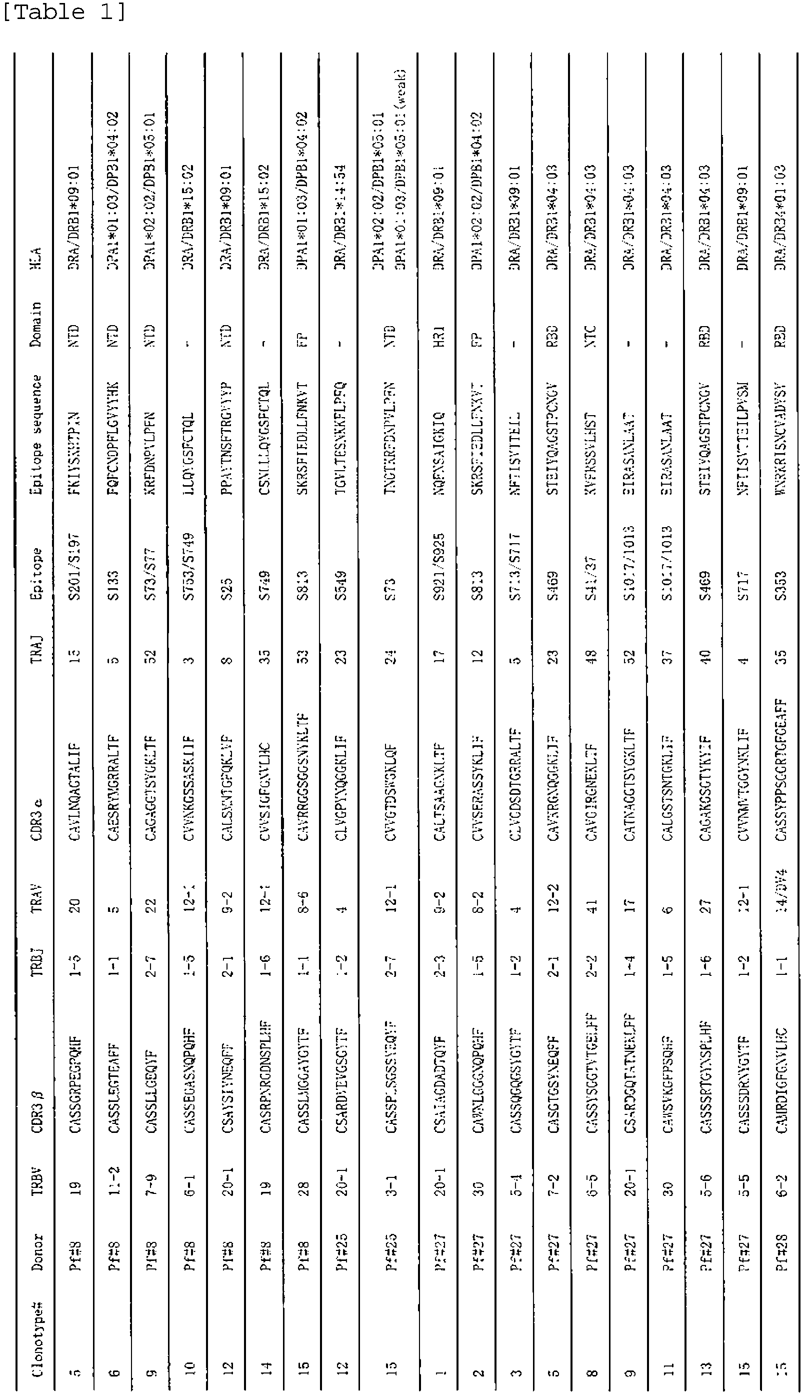

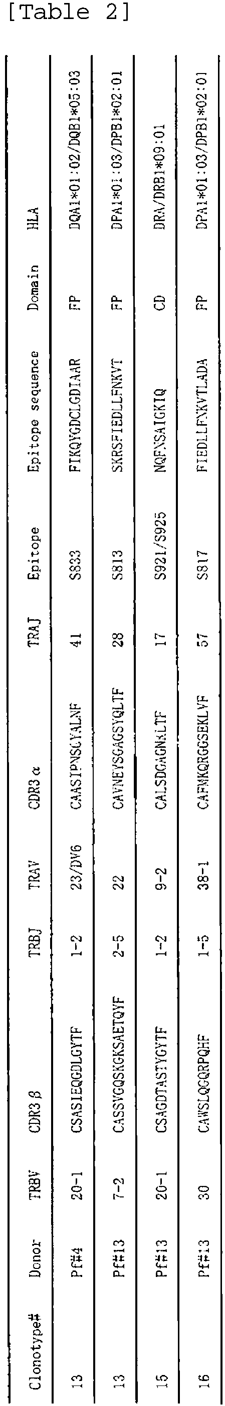

### [Experimental Example 2]

### (Material and method)

### Sample Collection

Seven-week-old BALB/cA mice (CLEA Japan, Inc., N=30) were used as experimental animals. The following partial peptides (Tfh epitopes) of the SARS-CoV-2 spike protein were used for vaccination.

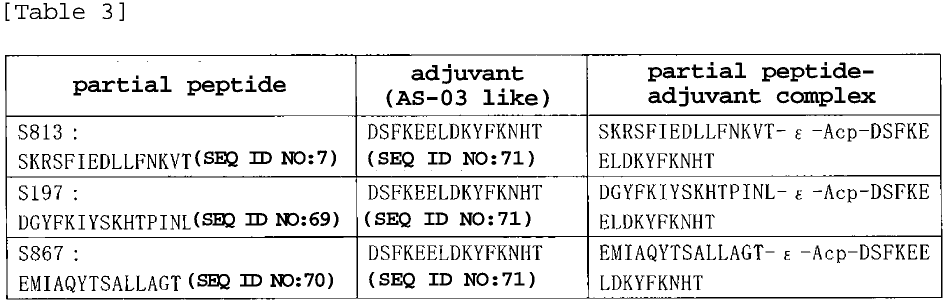

The partial peptides were administered at the doses shown in Table 4.

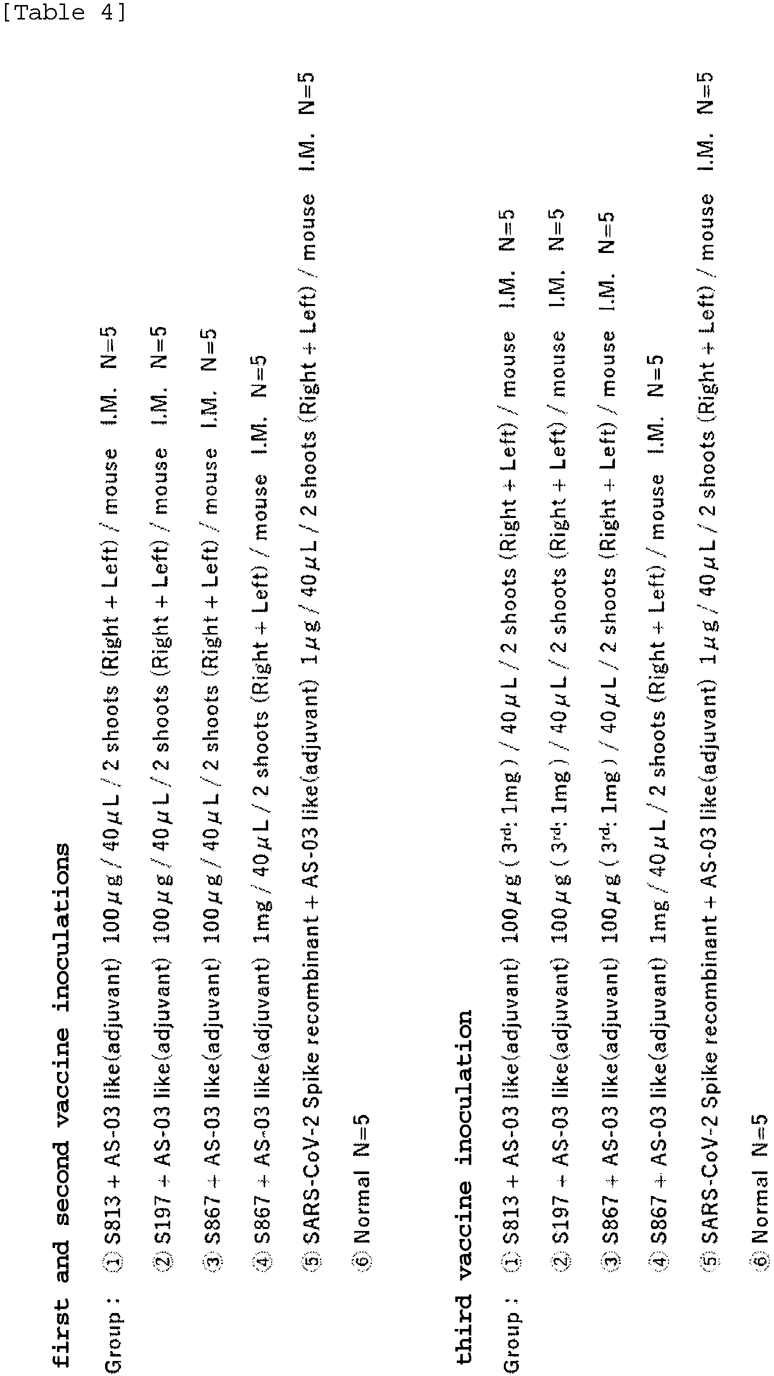

The vaccination schedule is shown in Table 5. Briefly, the first blood samples were collected before the first vaccination, then blood samples were collected at weeks 2, 4, 6, 8, 10, 12, and 14, and thereafter every four weeks. The first vaccination was performed immediately after the first blood sample collection, the second vaccination was performed two weeks later, and the third vaccination was performed within the six-week period.

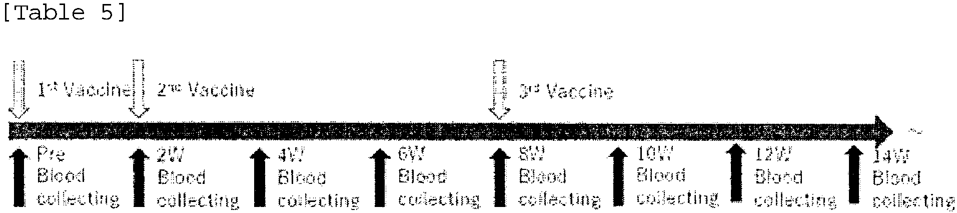

### Mouse antibody titer measurement by enzyme-linked immunosorbent assay (ELISA)

To quantify antibody production after vaccine injection, an ELISA assay was used. Briefly, recombinant precursor spike protein (S1+S2, Cell Signaling Technology; 1 µg/ml), and conjugation of antigen peptide and BSA (synthesized in PEPTIDE INSTITUTE, INC.; 10 µg/ml) were coated onto 96-well plates overnight at 4°C. On day 2, wells were blocked with a blocking buffer (PBS-T (0.05%) Tween 20, containing 5% skim milk) for 2 hr at room temperature. Serum was diluted 10- to 31,250-fold with the blocking buffer and incubated in the wells overnight at 4°C. The next day, the wells were washed and incubated with horseradish peroxidase (HRP)-conjugated antibody (GE Healthcare) for 3 hr at room temperature. After washing the wells with PBS-T, the wells were incubated with the peroxidase chromogenic substrate 3,3'-5,5'-tetramethyl benzidine (Sigma-Aldrich) for 30 min at room temperature, and the reaction was quenched by adding 0.5N sulfuric acid (Sigma-Aldrich). The absorbance of the wells was immediately measured at 450 nm using a microplate reader (Bio-Rad). The half-maximum antibody titer for each sample was calculated from the highest absorbance within the dilution range using Prism 8 software.

### (Results)

The results are shown in Fig. 12-1 to 12-5. It was shown that the partial peptide containing the amino acid sequences shown in SEQ ID NO: 69 and SEQ ID NO: 70 have the effect of increasing the mouse antibody titer.

### [Industrial Applicability]

According to the present invention, a vaccine for inducing a follicular helper T cell reactive to SARS-CoV-2 can be provided, which contains a SARS-CoV-2-derived specific peptide found from PBMC of a vaccinated patient. The SARS-CoV-2-derived specific peptide contained in the above-mentioned vaccine actually binds to the TCR of follicular helper T cells amplified in patients who received mRNA vaccine. Thus, the administration of the peptide strongly induces follicular helper T cells. The increase in the number of follicular helper T cells contributes to the maintenance of antibody production, making it possible to induce long-term immunity.

## Claims

1. A partial peptide of the spike protein of SARS-CoV-2, which comprises an amino acid sequence selected from the group consisting of the following (1) to (17) and has a full length of 15 or less amino acids:
(1) FKIYSKHTPIN (SEQ ID NO: 1),
(2) FQFCNDPFLGVYYHK (SEQ ID NO: 2),
(3) KRFDNPVLPFN (SEQ ID NO: 3),
(4) LLQYGSFCTQL (SEQ ID NO: 4),
(5) PPAYTNSFTRGVYYP (SEQ ID NO: 5),
(6) CSNLLLQYGSFCTQL (SEQ ID NO: 6),
(7) SKRSFIEDLLFNKVT (SEQ ID NO: 7),
(8) TGVLTESNKKFLPFQ (SEQ ID NO: 8),
(9) TNGTKRFDNPVLPFN (SEQ ID NO: 9),
(10) NQFNSAIGKIQ (SEQ ID NO: 10),
(11) NFTISVTTEIL (SEQ ID NO: 11),
(12) STEIYQAGSTPCNGV (SEQ ID NO: 12),
(13) KVFRSSVLHST (SEQ ID NO: 13),
(14) EIRASANLAAT (SEQ ID NO: 14),
(15) NFTISVTTEILPVSM (SEQ ID NO: 15),
(16) FIKQYGDCLGDIAAR (SEQ ID NO: 16), and
(17) FIEDLLFNKVTLADA (SEQ ID NO: 17).

2. A vaccine comprising the partial peptide according to claim 1.

3. The vaccine according to claim 2, for inducing a follicular helper T cell reactive to SARS-CoV-2.

4. The vaccine according to claim 3, wherein the SARS-CoV-2 is a strain selected from Wuhan strain, Delta strain, and Omicron strain.

5. The vaccine according to any one of claims 2 to 4, which is administered to a subject selected from the group consisting of the following (1) to (17):
(1) a subject of administration having DRB1*09:01 as an HLA gene capable of presenting a partial peptide of the spike protein of SARS-CoV-2, which peptide comprises the amino acid sequence of FKIYSKHTPIN (SEQ ID NO: 1) and has a full length of 15 or less amino acids,
(2) a subject of administration having DPA1*01:03/DPB1*04:02 as HLA genes capable of presenting a partial peptide of the spike protein of SARS-CoV-2, which peptide comprises the amino acid sequence of FQFCNDPFLGVYYHK (SEQ ID NO: 2) and has a full length of 15 or less amino acids,
(3) a subject of administration having DPA1*02:02/DPB1*05:01 as an HLA gene capable of presenting a partial peptide of the spike protein of SARS-CoV-2, which peptide comprises the amino acid sequence of KRFDNPVLPFN (SEQ ID NO: 3) and has a full length of 15 or less amino acids,
(4) a subject of administration having DRB1*15:02 as an HLA gene capable of presenting a partial peptide of the spike protein of SARS-CoV-2, which peptide comprises the amino acid sequence of LLQYGSFCTQL (SEQ ID NO: 4) and has a full length of 15 or less amino acids,
(5) a subject of administration having DRB1*09:01 as an HLA gene capable of presenting a partial peptide of the spike protein of SARS-CoV-2, which peptide comprises the amino acid sequence of PPAYTNSFTRGVYYP (SEQ ID NO: 5) and has a full length of 15 or less amino acids,
(6) a subject of administration having DRB1*15:02 as an HLA gene capable of presenting a partial peptide of the spike protein of SARS-CoV-2, which peptide comprises the amino acid sequence of CSNLLLQYGSFCTQL (SEQ ID NO: 6) and has a full length of 15 or less amino acids,
(7) a subject of administration having DPA1*01:03/DPB1*04:02, DPA1*02:02/DPB1*04:02 or DPA1*01:03/DPB1*02:01 as HLA genes capable of presenting a partial peptide of the spike protein of SARS-CoV-2, which peptide comprises the amino acid sequence of SKRSFIEDLLFNKVT (SEQ ID NO: 7) and has a full length of 15 or less amino acids,
(8) a subject of administration having DRB1*14:54 as an HLA gene capable of presenting a partial peptide of the spike protein of SARS-CoV-2, which peptide comprises the amino acid sequence of TGVLTESNKKFLPFQ (SEQ ID NO: 8) and has a full length of 15 or less amino acids,
(9) a subject of administration having DPA1*02:02/DPB1*05:01 or DPA1*01:03/DPB1*05:01 as HLA genes capable of presenting a partial peptide of the spike protein of SARS-CoV-2, which peptide comprises the amino acid sequence of TNGTKRFDNPVLPFN (SEQ ID NO: 9) and has a full length of 15 or less amino acids,
(10) a subject of administration having DRB1*09:01 as an HLA gene capable of presenting a partial peptide of the spike protein of SARS-CoV-2, which peptide comprises the amino acid sequence of NQFNSAIGKIQ (SEQ ID NO: 10) and has a full length of 15 or less amino acids,
(11) a subject of administration having DRB1*09:01 as an HLA gene capable of presenting a partial peptide of the spike protein of SARS-CoV-2, which peptide comprises the amino acid sequence of NFTISVTTEIL (SEQ ID NO: 11) and has a full length of 15 or less amino acids,
(12) a subject of administration having DRB1*04:03 as an HLA gene capable of presenting a partial peptide of the spike protein of SARS-CoV-2, which peptide comprises the amino acid sequence of STEIYQAGSTPCNGV (SEQ ID NO: 12) and has a full length of 15 or less amino acids,
(13) a subject of administration having DRB1*04:03 as an HLA gene capable of presenting a partial peptide of the spike protein of SARS-CoV-2, which peptide comprises the amino acid sequence of KVFRSSVLHST (SEQ ID NO: 13) and has a full length of 15 or less amino acids,
(14) a subject of administration having DRB1*04:03 as an HLA gene capable of presenting a partial peptide of the spike protein of SARS-CoV-2, which peptide comprises the amino acid sequence of EIRASANLAAT (SEQ ID NO: 14) and has a full length of 15 or less amino acids,
(15) a subject of administration having DRB1*09:01 as an HLA gene capable of presenting a partial peptide of the spike protein of SARS-CoV-2, which peptide comprises the amino acid sequence of NFTISVTTEILPVSM (SEQ ID NO: 15) and has a full length of 15 or less amino acids,
(16) a subject of administration having DQA1*01:02/DQB1*05:03 as an HLA gene capable of presenting a partial peptide of the spike protein of SARS-CoV-2, which peptide comprises the amino acid sequence of FIKQYGDCLGDIAAR (SEQ ID NO: 16) and has a full length of 15 or less amino acids, and
(17) a subject of administration having DPA1*01:03/DPB1*02:01 as HLA genes capable of presenting a partial peptide of the spike protein of SARS-CoV-2, which peptide comprises the amino acid sequence of FIEDLLFNKVTLADA (SEQ ID NO: 17) and has a full length of 15 or less amino acids.
